# EUROPEAN PATENT SPECIFICATION

(11) **EP 0 464 175 B1**
(45) Date of publication and mention of the grant of the patent: **07.01.1998**
(21) Application number: 91902691.4
(22) Date of filing: 21.01.1991
(51) Int. Cl.: C12N 15/12, C07K 14/71, A61K 47/00

(54) **EXTRACELLULAR FORM OF THE HUMAN FIBROBLAST GROWTH FACTOR RECEPTOR**
EXTRAZELLULÄRE FORM DES MENSCHLICHEN FIBROBLASTEN WACHSTUMSFAKTORREZEPTORS
FORME EXTRACELLULAIRE DU RECEPTEUR DE CROISSANCE FIBROBLASTIQUE HUMAIN

(30) Priority: 23.01.1990 GB 9001466
(43) Date of publication of application: 08.01.1992
(73) Proprietor: PHARMACIA & UPJOHN S.p.A., 20152 Milano (IT)
(72) Inventor: BERGONZONI, Laura, I-20161 Milan (IT); MAZUE, Guy, I-20100 Milan (IT); ISACCHI, Antonella, I-20100 Milan (IT); RONCUCCI, Romeo, I-20100 Milan (IT); SARMIENTOS, Paolo, I-20100 Milan (IT)
(74) Representative: Woods, Geoffrey Corlett
(86) International application number: EP9100103
(87) International publication number: WO9111459

(56) References cited:
- WO-A-91/00916
- Science, volume 245, 7 July 1989; P.L. Lee et al.: "Purification and complementary DNA cloning of a receptor for basic fibroblast growth factor", pages 57-60
- Proc. Natl. Acad. Sci., volume 86, July 1989, E.B. Pasquale et al.: "Identification of a developmetally regulated proteintyrosine kinase by using anti-phos-photyrosine antibodies to screen a cDNA expression library", pages 5449-5453
- Biochemical and Biophysical Research Communications, volume 169, no. 2, Academic Press Inc., pages 680-685; N. Itoh et al
- Nucleic Acids Research, volume 18, no. 7, 18 November 1990, Oxford University Press, (Eynsham, Oxford, GB); A. Isacchi et al.: "Complete sequence of a human receptor for acidic and basic fibroblast growth factors", page 2616
- Oncogene, vol. 3, 1988, pages 9-15; Ruta, M. et al

## Description

The present invention relates to human growth factor receptors.

The formation of blood capillaries occurs in a number of important biological processes, either physiological, such as organ development and wound healing, or pathological, such as tumor growth. While the sequence of events leading to neovascularization has been characterized morphologically, the molecular mechanisms by which this process occurs are still poorly understood. The control of growth in the capillary endothelium appears to be very tightly controlled, since these cells normally form a static monolayer whose proliferation is triggered in the angiogenic process. The normally quiescent nature of the endothelial cells may be explained in part by the apparent lack of endothelial cell growth factors to plasma. The major endothelial cell mitogens in fact are not found in plasma, although they are present in extracts of almost all tissues studied and in many normal and tumor cell lines as well. Therefore, the localized induction of rapid endothelial cell proliferation may involve the release of endothelial cell mitogens from cells in response to environmental cues.

The best characterized of the endothelial cell mitogens are a family of polypeptide growth factors, including basic fibroblast growth factor (bFGF), also known as heparin-binding growth factors for their high affinity to heparin. Basic FGF has been purified from most mesoderm- or neuroectoderm-derived tissues or cells. Structural studies have shown that bFGF is a single chain polypeptide made of 146 amino acids, which can also exist in NH₂-terminally truncated forms missing the first 10-20 amino acids. The truncated forms of bFGF are as potent as native bFGF, as demonstrated by radioreceptor binding and biological assays. In addition, modifications of the purification protocols by substitution of neutral for acidic extraction from homogeneized tissue and inclusion of protease inhibitors have yielded a longer 154-residue form. The observed microheterogeneity of FGFs seems to be due, at least in part, to partial proteolysis near the amino termini that occurs either in vivo or during purification. However, because the various forms appear to be equally active, the microheterogeneity is probably physiologically, irrelevant.

Basic FGF seems to have been extremely well conserved through evolution. For example, bovine and human bFGF differ in only two of their 146 amino acids, giving an overall amino acid sequence homology of 98.7%. Related to bFGF is acidic FGF (aFGF), which shares a 55% total sequence homology with bFGF. Acidic FGF is a 140-amino acid polypeptide that can also exist in a NH₂-terminally truncated form missing the first 6 amino acids. As expected from their high degree of homology, both basic and acidic FGF seem to interact with the same cell-surface receptors. This explains their common range of target cells and spectrum of biological activity.

Recently, Lee et al (Science, 245, 57-60, 1989) have described the purification, from chicken embryos, of a new membrane protein able to bind specifically basic FGF. On the base of its biochemical characteristics and on homology with other known receptors, this new protein is thought to be a basic FGF receptor. In the same article the authors describe the isolation of a chicken full-length cDNA clone coding for the described protein. The nucleotide sequence of this clone is however not disclosed.

Lee et al noted that the chicken bFGF receptor shows a considerable amino acid similarity to a previously identified human polypeptide sequence which is the product of the flg gene (Ruta et al, Oncogene, 3, 9-15, 1988). Like the chicken bFGF receptor, the flg molecule seems to be a tyrosine kinase, shows a typical hydrophobic transmembrane region and consequently it is likely that the described human sequence could be part of the human bFGF receptor.

The amino acid sequence of the flg molecule was deduced by Ruta et al by the translation of an open reading frame of a partial cDNA clone. The flg cDNA sequence, according to Ruta et al was obtained by low-stringency screening of a human endothelial cell cDNA library using as probe a DNA fragment coding for a tyrosine kinase oncogene. Nevertheless, the flg cDNA clone, described to date, is only partial and lacks the nucleotide sequence coding for the extracellular portion of the human bFGF receptor.

WO 91/00916, which was not published until after the present application was filed but which has an earlier claimed priority date than the present application, discloses the cDNA and amino acid sequences of a human FGF receptor that is different from the receptor which is the subject of the present application.

In summary, the only amino acid or nucleotide sequences published prior to the present invention were those corresponding to the complete chicken bFGF receptor and to a portion of the human basic FGF receptor. The complete extracellular amino acid sequence of the human bFGF receptor, which is responsible for the specific binding of human basic and acidic FGFs, was unpublished.

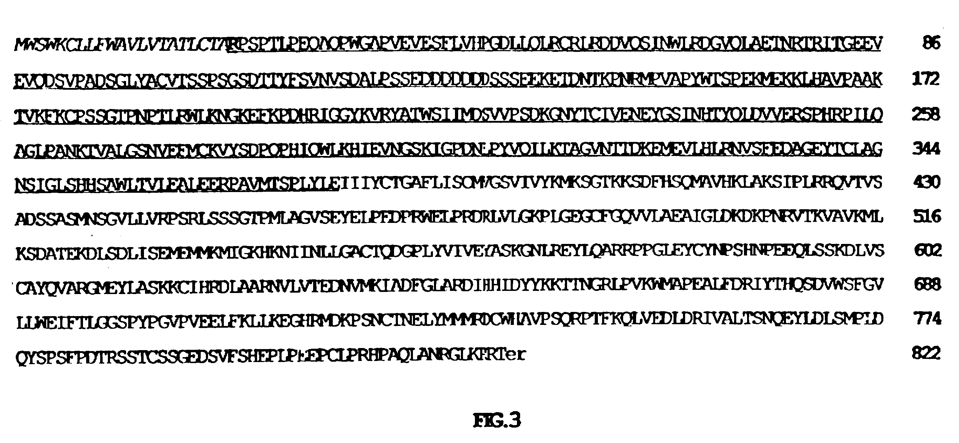

We have now cloned a human bFGF receptor and have identified its extracellular portion. The extracellular portion may be used an as antagonist for bFGF or aFGF. Accordingly, the present invention provides a polypeptide which is capable of binding specifically human basic Fibroblast Growth Factor (bFGF) and human acidic Fibroblast Growth Factor (aFGF) and which has:
(a) the sequence underlined in Figure 3, or
(b) said sequence (a) modified by an amino acid extension sequence at either or each end.

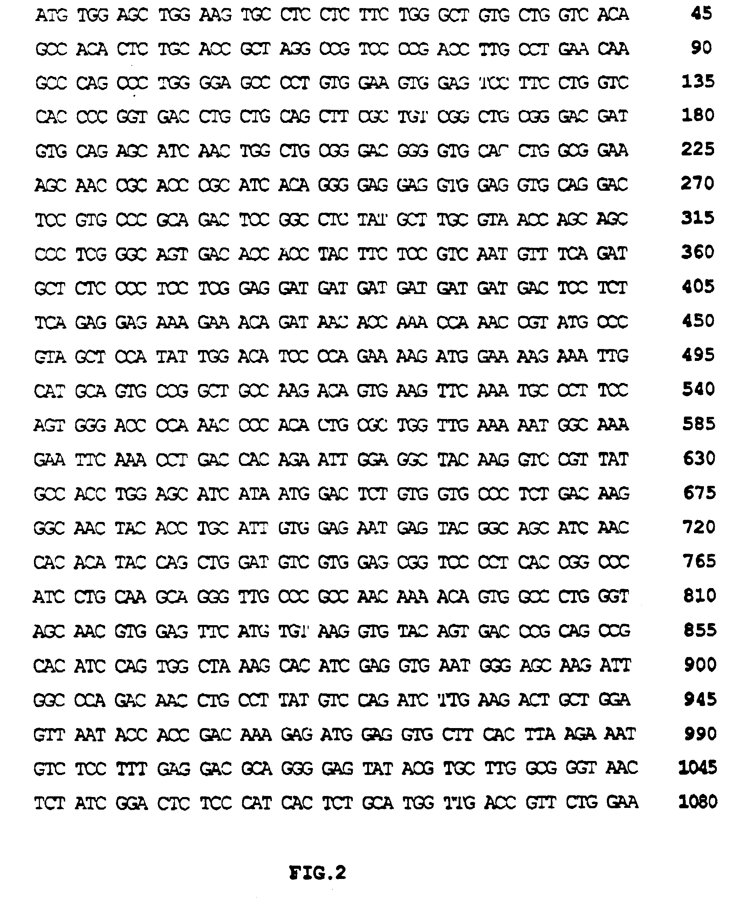

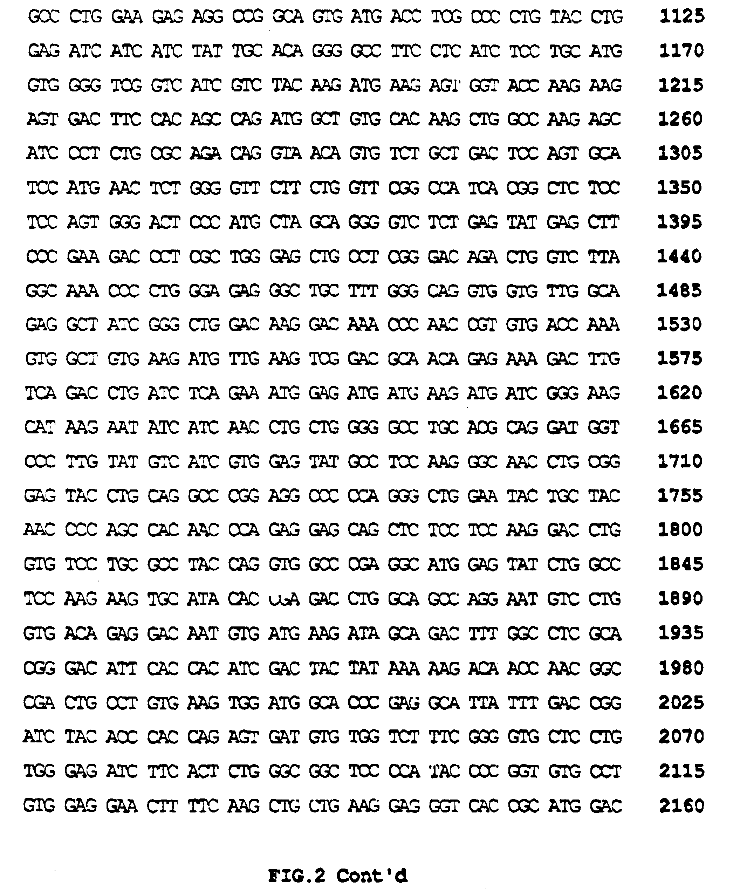

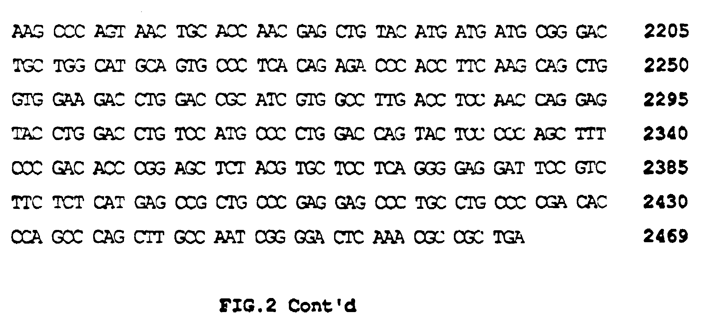

The invention also provides a DNA molecule which encodes a polypeptide of the invention. The DNA molecule may have the sequence from nucleotide 64 to nucleotide 1128 shown in Figure 2.

The invention further provides a vector which incorporates a DNA molecule of the invention and which is capable, when provided in a transformed host, of expressing the polypeptide of the invention encoded by the DNA molecule. A host transformed with such a vector forms part of the present invention too.

A polypeptide of the invention is prepared by a process which comprises culturing a transformed host according to the invention under such conditions that the polypeptide is expressed. The polypeptide can then be isolated. The polypeptide may be recovered in biologically pure form.

In the accompanying drawings:
Figure 1 is a schematic illustration of clones PL5 and PL10;
Figure 2 shows the nucleotide sequence of the bFGF receptor molecule; and
Figure 3 shows the amino acid sequence of the bFGF receptor. The putative leader peptide is in italic characters. The previously unknown extracellular portion is underlined. The arginine (R) residue which is thought to be the first amino acid of the mature molecule is in a bold character.
Figure 4 shows the two intermediate constructions (plasmids L23 and pFC42) as well as the two final expression plasmids (pFC138 and pFC164) carrying the sequence coding for the extracellular form of the FGF receptor. For details, see Example 2 below.

Polypeptides of the invention have the sequence underlined in Figure 3 from amino acid 22 to amino acid 376. This is the sequence of the extracellular portion of the bFGF receptor without the putative leader sequence.

The sequence underlined in Figure 3 may be modified by one or more amino acid substitutions and/or insertions. A polypeptide composed of such a modified sequence must of course still be capable of binding human bFGF and aFGF. For a suitable receptor-binding test, see Dower et al, J. Immunol. 42, 4314-4320, 1989. When the underlined sequence shown in Figure 3 is modified, there is a degree of homology of 85% or more between the modified sequence and the unmodified sequence. The degree of homology may be 95% or more.

For example, one or more amino acid residues of the sequence underlined in Figure 3 may be substituted or one or more additional amino acid residues may be inserted; provided the physicochemical character of the original sequence is preserved, i.e. in terms of charge density, hydrophobicity/hydrophilicity, size and configuration. Candidate substitutions are, based on the one-letter code (Eur. J. Biochem. 138, 9-37, 1984):
A for G and vice versa
V by A, L or G;
K by R;
S by T and vice versa;
E for D and vice versa; and
Q by N and vice versa.

As far as extensions are concerned, a short sequence of up to 50 amino acid residues may be provided at either or each terminal. The sequence may have up to 30, for example up to 20 or up to 10, amino acid residues. Alternatively, a much longer extension may be present. Longer amino acid sequences may be fused to either or each end. A chimaeric protein may therefore be provided in which the or each extension is a heterologous amino acid sequence, i.e. a sequence not naturally linked to the sequence derived from Figure 3. Such a chimaeric protein may therefore combine the ability to bind specifically to bFGF and aFGF with another functionality.

The polypeptides are prepared by recombinant DNA technology. The preparation of the polypeptides therefore depends upon the provision of a DNA sequence encoding the polypeptide. DNA having the sequence shown in Figure 2 may be obtained by probing a human placenta cDNA library, for example a Xgtll library. Such a library is available from Clontech. Suitable probes are: ATAACGGACCTTGTAGCCTCCAATTCTGTG, GGGTCTCTGTGAGGGCACTGCATGCCAGCA, and

ACGGCCTAGCGGTGCAGAGTGTGGCTGTGA.

A shorter sequence than that shown in Figure 2 may be obtained by use of restriction endonucleases and/or exonucleases. A DNA sequence from nucleotide 64 to nucleotide 1128 may be provided. Modified sequences may be obtained by use of any appropriate technique, including restriction with an endonuclease, insertion of linkers, use of an exonuclease and/or a polymerase and site-directed mutagenesis. Whether a shortened and/or modified DNA sequence encodes a polypeptide of the invention can be readily ascertained. The polypeptide encoded by the sequence can be expressed in a suitable host and tested for its ability to bind specifically human bFGF and aFGF.

For expression of a polypeptide of the invention, an expression vector is constructed. An expression vector is prepared which comprises a DNA sequence encoding a polypeptide of the invention and which is capable of expressing the polypeptide when provided in a suitable host. Appropriate transcriptional and translational control elements are provided, including a promoter for the DNA sequence, a transcriptional termination site, and translational start and stop codons. The DNA sequence is provided in the correct frame such as to enable expression of the polypeptide to occur in a host compatible with the vector.

The expression vector is then provided in an appropriate host. Cells harbouring the vector are grown so as to enable expression to occur. The vector may be a plasmid or a viral vector. Any appropriate host-vector system may be employed.

The transformed host may be a prokaryotic or eukaryotic host. A bacterial or yeast host may be employed, for example E. coli or S. cerevisiae. Insect cells can alternatively be used, in which case a baculovirus expression system may be appropriate. As a further alternative, cells of a mammalian cell line, such as Chinese Hamster Overy (CHO) cells may be transformed.

The polypeptide of the invention can be isolated and purified. The polypeptide can be employed as a human bFGF or aFGF antagonist. It may sequester bFGF in vivo, thus preventing the biological activity of bFGF and acting as an inhibitor of bFGF activities. Antagonists of bFGF and aFGF activities could have clinical applications in several pathologies related to abnormal angiogenesis such as diabetic, retinopathy, neovascular glaucoma, rheumatoid arthritis, psoriasis, artherosclerosis and as contraceptives. In addition, the observation that certain solid tumors require neovascularization to grow suggests that FGF antagonists could be therapeutically developed to treat these diseases.

For this purpose, a polypeptide of the present invention may be chemically coupled to another material to provide a conjugate. The other material may be a carrier molecule or a molecule with another biological function. The polypeptide of the invention may also be formulated in a pharmaceutical composition. The pharmaceutical composition also comprises a pharmaceutically acceptable carrier or diluent. The pharmaceutical composition may contain the polypeptide of the invention in the form of a conjugate.

The polypeptide of the invention may be administered to a patient by any convenient route. The choice of whether an oral route or a parenteral route, such as subcutaneous, intravenous or intramuscular administration, is adopted; of the dose; and of the frequency of administration depends upon a variety of factors. These factors include the purpose of the administration, the age and weight of the patient being treated and the condition of the patient. Typically, however, the polypeptide is administered in an amount of from 1 to 1000 µg per dose, more preferably from 10 to 100 µg per dose, for each route of administration.

The following Example illustrates the invention.

### EXAMPLE 1

A cDNA clone coding for basic FGF receptor was isolated by screening a human placenta λgt11 cDNA library. This library is commercially available from Clontech.

A partial cDNA clone thought to encode a portion of the human bFGF receptor was already published in the literature (Ruta et al, 1988). According to this article, we designed two oligonucleotide probes with the following sequences: 5' ATAACGGACCTTGTAGCCTCCAATTCTGTG 3' (named OAB965) and

5' GGGTCTCTGTGAGGGCACTGCATGCCAGCA 3' (named OAB984).

A first screening was carried out using only the probe OAB965. The oligonucleotide was kinased using (γ³²P)ATP and purified on a Nensorb column (Nen). Following the determination of the cDNA library titre (2 x 10⁹ pfu/ml), appropriate dilutions were made and plated using the E. coli strain Y1090. 0.05 ml of an overnight culture of Y1090 were mixed with 0.1 ml of sterile lambda diluent (10 mM Tris HCl pH 7.5, 10 mM MgCl₂, 0.1 mM EDTA) containing a 4 x 10⁻³ dilution of the library and incubated at 37°C for 15 minutes to allow phage adsorption. Plating was carried out on 20 Petri dishes of LB containing ampicillin 100 µg/ml.

According to published procedures (Maniatis et al, Molecular cloning: a laboratory manual, Cold Spring Harbor Laboratory, Cold Spring Harbor, New York 1982) 10⁶ plaques were transferred onto nitrocellulose filters and hybridized under low stringency conditions (5X SSC, 0.1% SDS, 5X Denhardt solution, 0.1 mg/ml salmon sperm DNA), overnight at 65°C. The labelled probe was added at 10⁶ cpm/ml. Washes were performed at 65°C in 6X SSC, 0.1% SDS and in 3X SSC, 0.1% SDS. Three positive clones, named PL3, PL10, and PL16 were detected after an overnight exposure with Emersham Hyperfilm -MP. These positive signals were confirmed in a separate screening using the same probe.

A second screening was then performed on PL3, PL10 and PL16 on duplicate filters using the two probes OAB 965 and OAB 984. Interestingly, these clones were recognized by both probes.

The three clones were plaque-purified and further analyzed. Phage DNA of PL3, PL10, and PL16 was purified using Lambdasorb phage adsorbent (Promega) and digested with EcoRI. From the different digestions, we selected two inserts of 2.8 Kb and 0.6 Kb from PL10 and subcloned them into M13mp19.

Nucleotide sequence analysis was carried out by primed DNA synthesis on single-stranded DNA templates in the presence of dideoxynucleoside triphosphates (Sanger et al, Proc. Natl. Acad. Sci. USA 74, 5463-5467, 1977) by using Sequenase (United States Biochemical Corp. Cleveland, Ohio). Both strands of the subclones were sequenced using either universal or specific primer oligonucleotides based on the generated sequence.

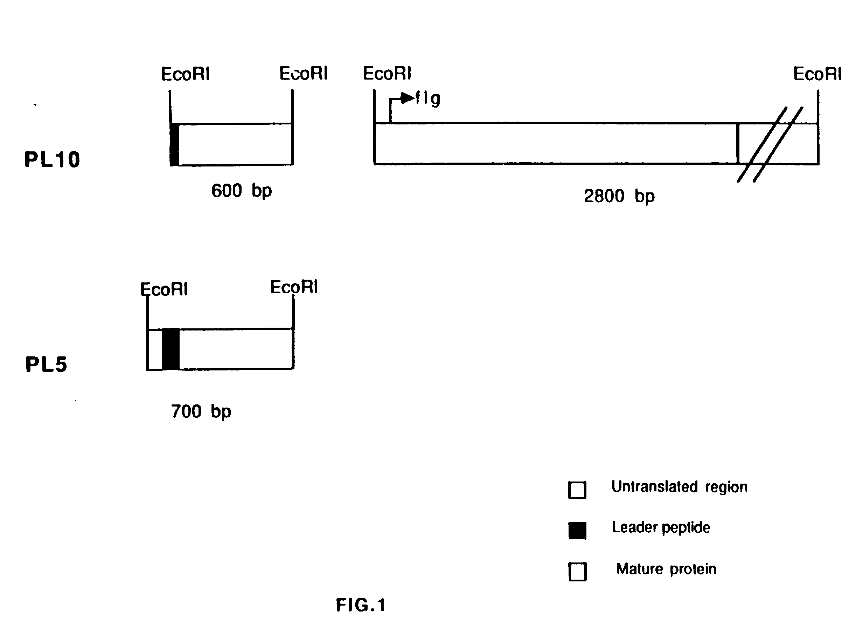

A single open reading frame for 817 amino acids with a termination codon could be detected. The first 196 amino acids of the mature bFGF receptor are codified by the insert of 0.6 Kb. The final sequence of this fragment contains an EcoRI site, so that the 626 amino acids of the COOH-terminus are codified by the 2.8 Kb insert and followed by a 3' end untranslated region, as schematically illustrated in Fig. 1.

The chicken receptor for bFGF, as it has been described by Lee et al (1989), is a protein of 819 amino acids including a leader peptide of 21 amino acids, followed by a mature protein of 798 amino acids. By homology to this sequence, our clone contained the complete mature bFGF receptor molecule, preceded by 16 amino acids of the leader peptide. In order to find the remaining 5 amino acids of the leader peptide, including the starting methionine, we synthesized a new oligonucleotide probe with the following sequence, derived from the 0.6 kb insert of PL10:

5′ ACGGCCTAGCGGTGCAGAGTGTGGCTGTGA 3′ (named OAB 1088)

By screening the library with this probe, we selected a new clone, name PL5, which contained a 0.7 kb EcoRI fragment. This fragment was sequenced and found to contain the 0.6 kb sequence from PL10, while it further extended at the 5' end. More precisely, the 0.7 kb sequence from PL5 codes for the 196 amino acids of the NH₂ terminal sequence, also found in PL10, preceded by 21 amino acids of the leader peptide, starting with a methionine. A schematic description of clones PL5 and PL10 is illustrated in Fig. 1.

The full length DNA sequence for a bFGF receptor, as obtained from the two overlapping clones PL5 and PL10, is depicted in Figure 2. The corresponding amino acid sequence is depicted in Figure 3. In this sequence, the putative leader peptide of 21 amino acids is in italics. The arginine (R) residue which is thought to be the first amino acid of the mature molecule is in bold character. The previously unknown extracellular portion is underlined.

The full length clone and the extracellular domain have been expressed in both mammalian and bacterial cells. Cross-linking experiments with labelled basic and acidic FGFs have shown that the recombinant complete receptor and extracellular portion are indeed able to bind specifically basic and acidic FGF.

### EXAMPLE 2

To obtain the expression of the extracellular portion of the human FGF receptor in *E. coli* a vector was constructed using as regulatory signals the tryptophan promoter of *E.coli* and the ribosome binding site region of the lambda CII protein (Hendrix R.W., Roberts J.W., Stahl F.W. and Weisberg R.A.: Lambda II Cold Spring Harbour Laboratory, Cold Spring Harbour, N.Y. 1983).

The 5' end of the receptor sequence was synthetically reconstructed to be cloned into the expression vector. For this purpose a HindIII-PstI synthetic DNA fragment coding for the CII ribosome binding site followed by the ATG codon and the first 95 nucleotides of the mature receptor sequence (Fig.2: nucleotides 61 to 155) was synthesized using complementary oligonucleotides. The complete sequence of such synthetic fragment is:

5' AGCTTGGGCATACATTCAATCAATTGTTATCTAAGGAAATACTTAC
ATATGGCTAGGCCGTCCCCGACCTTGCCTGAACAAGCCCAGCCCTGGGG
AGCCCCTGTGGAAGTGGAGTCCTTCCTGGTCCACCCCGGTGACCTGCT
GCA 3'

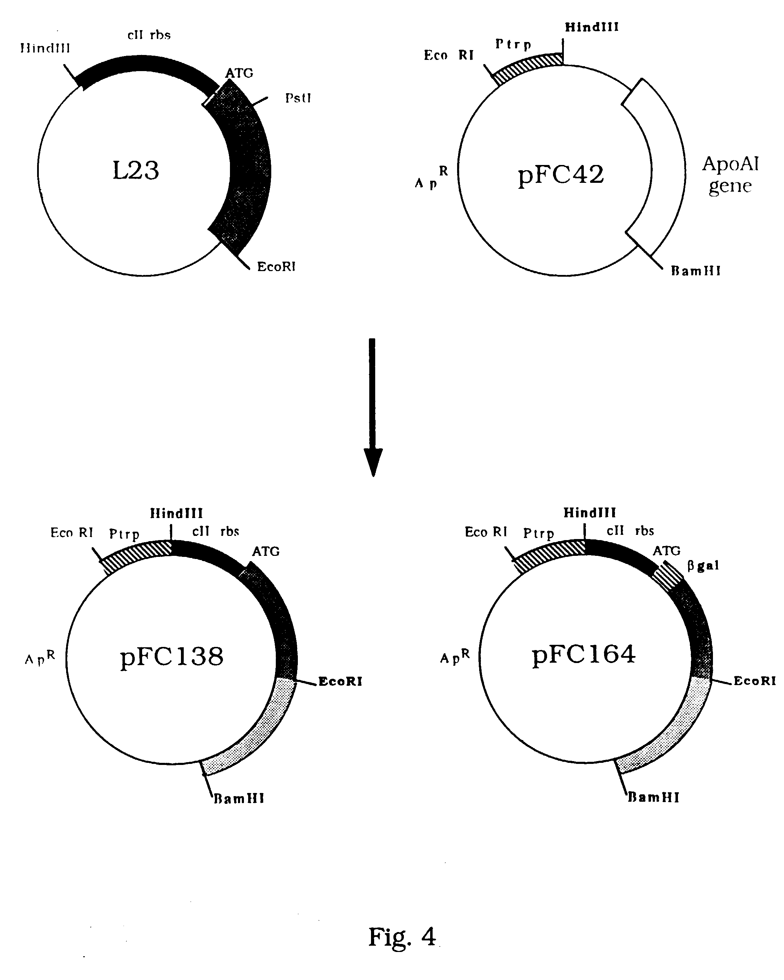

This HindIII-PstI fragment was subcloned into M13mp18 previously cut with HindIII and EcoRI, together with a PstI-EcoRI fragment from PL5, harbouring the receptor sequence corresponding to nucleotides 156 to 586 of fig.2. The resulting plasmid L23 carries the CII ribosome binding site followed by nucleotides 61 to 586 of the receptor sequence (See Fig.4), corresponding to the first 175 amino acids of the mature protein. To obtain the whole extracellular portion of the FGF receptor an EcoRI-BamHI fragment was recovered by polymerase chain reaction (PCR) on PL10. The reaction was performed in a Perkin Elmer DNA thermal cycler, using the Perkin Elmer Amplitaq DNA Polymerase and the following cycles: 2'30" at 94°C, 30 cycles made by one step of 1'30" at 94°C and a second step of 2'30" at 72°C and a final cycle of 7' at 72°C. The primers used for the reaction , called OAB 1192 and OAB 1195, were the following:

OAB 1192 : 5' GTAAAACGACGGCCAGT 3'

OAB 1195 : 5' ATGAGGATCCTCACTCCAGGTACAGGGGCGAGGTCA 3'

The amplified product was digested with EcoRI and BamHI to obtain a fragment of 546 bp, corresponding in fig.2 to nucleotides 587 to 1128 of the mature receptor sequence.

The expression plasmid for the mature extracellar receptor protein, named pFC138, was constructed by joining the following three fragments:
1) The HindIII-EcoRI fragment from L23.
2) The EcoRI-BamHI fragment from PL10.
3) The HindIII-BamHI large fragment from plasmid pFC42, previously obtained in our laboratory (Isacchi A., Sarmientos P., Lorenzetti R. and Soria M.: Mature apolipoprotein AI and its precursor proApo AI: influence of the sequence at the 5' end of the gene on the efficiency of expression in *Eschedchia coli.* Gene 81 p.129-137 1989), harbouring the tryptophan promoter and the β-lactamase gene for resistance to ampicillin.

An *E.coli* B strain ( Delbruck M.: Bacterial viruses or bacteriophages. Biol.Rev. 21 p.30-40 1946) was used for expression of the mature extracellular FGF receptor protein.

Induction and analysis of protein expression were performed as described (Isacchi A. et al.).

The protein was recognized in western blot by rabbit anti-human FGF receptor polyclonal antibodies (Promega).

Alternatively, the recombinant extracellular domain could be synthesized as a fusion protein where additional amino acid residues are present either at the NH₂- end or at the COOH-end of the mature extracellular region.

This approach is currently used for increasing intracellular protein stability, yield, detection and isolation.

Both patent and scientific literature report many examples of different expression systems based on fusion proteins [see, for example, International Patent Application PCT/WO84/00380; European Patent Application EP 243333; Harris, T.J.R. 1983, Expression of eukaryotic genes in E. coli, pp. 127-185. In Williamson, R. (Ed.), Genetic Engineering Vol. 4, Academic Press, London; Hellebust, M. et al. 1989, Biotechnology Vol. 7, pp 165-168].

We have constructed a new plasmid named pFC 164, shown in figure 4, wherein the amino acids 9 to 23 of the E. coli β-galactosidase are inserted upstream of the mature extracellular domain of the FGF receptor. Plasmid pFC 164 is constructed in a way similar to pFC 138 and when introduced in the E. coli host strain B, yields higher levels of expression of the extracellular domain of the FGF receptor. The β-galactosidase moiety is also responsible for an increased intracellular stability of the fusion protein.

Plasmids pFC 138 and pFC 164 are therefore objects of the present invention.

## Claims

1. A polypeptide which is capable of binding specifically human basic Fibroblast Growth Factor (bFGF) and human acidic Fibroblast Growth Factor (aFGF) and which has:
(a) the sequence underlined in Figure 3, or
(b) said sequence (a) modified by an amino acid extension sequence at either or each end.

2. A polypeptide according to claim 1 wherein said amino acid extension sequence is a heterologous amino acid sequence.

3. A polypeptide according to claim 1 or 2 wherein said amino acid extension sequence has up to 50 amino acid residues.

4. A polypeptide according to claim 1, 2 or 3 modified in that the sequence underlined in Figure 3 has one or more amino acid substitutions and/or insertions, and wherein the modified sequence has a degree of homology of 85% or more with the sequence underlined in Figure 3.

5. A polypeptide according to claim 1, 2 or 3 modified in that the sequence underlined in Figure 3 has one or more amino acid substitutions and/or insertions, and wherein the modified sequence has a degree of homology of 95% or more with the sequence underlined in Figure 3.

6. A DNA molecule which encodes a polypeptide as defined in any one of claims 1 to 5.

7. A DNA molecule according to claim to 6, which has the sequence from nucleotide 64 to nucleotide 1128 shown in Figure 2.

8. A vector which incorporates a DNA molecule as claimed in claim 6 or 7 and which is capable, when provided in a suitable host, of expressing said polypeptide.

9. A vector according to claim 8, which is a plasmid.

10. The plasmid pFC 138 shown in Figure 4.

11. The plasmid pFC 164 shown in Figure 4.

12. A host transformed with a vector or a plasmid as claimed in any one of claims 8 to 11.

13. A host according to claim 12 which is a mammalian cell line.

14. A host according to claim 12 which is a bacterium.

15. A process for the preparation of a polypeptide as defined in any one of claims 1 to 5, which process comprises culturing a transformed host as claimed in any one of claims 12 to 14 under such conditions that the said polypeptide is expressed.

16. A pharmaceutical composition comprising a pharmaceutically acceptable carrier or diluent and, as an active principle, a polypeptide as claimed in any one of claims 1 to 5.

17. A polypeptide as defined in any one of claims 1 to 5 for use as an antagonist for human bFGF or human aFGF.

## Patentansprüche

1. Polypetid, das zur spezifischen Bindung von humanem basischen Fibroblastwachstumsfaktor (bFGF) und humanem sauren Fibroblastwachstumsfaktor (aFGF) in der Lage ist, und welches aufweist:
(a) die in Fig. 3 unterstrichene Sequenz, oder
(b) die Sequenz (a), welche durch eine Aminosäure-Verlängerungssequenz an einem oder beiden Enden modifiziert wurde.

2. Polypeptid nach Anspruch 1, worin die Aminosäure-Verlängerungssequenz eine heterologe Aminosäuresequenz ist.

3. Polypeptid nach Anspruch 1 oder 2, worin die Aminosäure-Verlängerungssequenz bis zu 50 Aminosäurereste aufweist.

4. Polypeptid nach Anspruch 1, 2 oder 3, das dahingehend modifiziert ist, daß die in Fig. 3 unterstrichene Sequenz eine oder mehrere Aminosäuresubstitutionen und/oder -insertionen aufweist und worin die modifizierte Sequenz einen Homologiegrad von 85 % oder mehr mit der in Fig. 3 unterstrichenen Sequenz hat.

5. Polypeptid nach Anspruch 1, 2 oder 3, das dahingehend modifiziert ist, daß die in Fig. 3 unterstrichene Sequenz eine oder mehrere Aminosäuresubstitutionen und/oder -insertionen aufweist und worin die modifizierte Sequenz einen Homologiegrad von 95 % oder mehr mit der in Fig. 3 unterstrichenen Sequenz aufweist.

6. DNA-Molekül, welches ein Polypeptid, wie in einem der Ansprüche 1 bis 5 definiert, kodiert.

7. DNA-Molekül, welches die Sequenz von Nukleotid 64 bis Nukleotid 1128, wie in Fig. 2 gezeigt, hat.

8. Vektor, welcher ein DNA-Molekül wie in Anspruch 6 oder 7 umfaßt, und welcher bei Bereitstellung in einem geeigneten Wirt zur Expression des Polypeptids in der Lage ist.

9. Vektor nach Anspruch 8, welcher ein Plasmid ist.

10. Plasmid pFC 138 wie in Fig. 4 gezeigt.

11. Plasmid pFC 164 wie in Fig. 4 gezeigt.

12. Wirt, transformiert mit einem Vektor oder einem Plasmid nach einem der Ansprüche 8 bis 11.

13. Wirt nach Anspruch 12, welcher eine Säugerzellinie ist.

14. Wirt nach Anspruch 12, welcher ein Bakterium ist.

15. Verfahren zur Herstellung eines Polypeptids nach einem der Ansprüche 1 bis 5, wobei das Verfahren die Kultur eines transformierten Wirts nach einem der Ansprüche 12 bis 14 unter solchen Bedingungen umfaßt, daß das Polypeptid exprimiert wird.

16. Pharmazeutische Zusammensetzung, umfassend einen pharmazeutisch verträglichen Träger oder Verdünner und, als Wirkprinzip ein Polypeptid nach einem der Ansprüche 1 bis 5.

17. Polypeptid nach einem der Ansprüche 1 bis 5 zur Verwendung als Antagonist für humanen bFGF oder humanen aFGF.

## Revendications

1. Polypeptide qui est capable de se lier de façon spécifique au Facteur de Croissance basique humain des Fibroblastes (bFGF) et au Facteur de Croissance acide humain des Fibroblastes (aFGF) et qui a :
(a) la séquence soulignée sur la Figure 3, ou
(b) ladite séquence (a) modifiée par une séquence d'extension d'acides aminés à l'une ou l'autre de ses extrémités ou à chacune de ces dernières.

2. Polypeptide selon la revendication 1, dans lequel ladite séquence d'extension d'acides aminés est une séquence d'acides aminés hétérologue.

3. Polypeptide selon la revendication 1 ou 2, dans lequel ladite séquence d'extension d'acides aminés a jusqu'à 50 restes d'acides aminés.

4. Polypeptide selon la revendication 1, 2 ou 3, modifié par le fait que la séquence soulignée sur la Figure 3 a une ou plusieurs substitutions et/ou insertions d'acides aminés, et dans lequel la séquence modifiée a un degré d'homologie de 85% ou davantage avec la séquence soulignée sur la Figure 3.

5. Polypeptide selon la revendication 1, 2 ou 3, modifié par le fait que la séquence soulignée sur la Figure 3 a une ou plusieurs substitutions et/ou insertions d'acides aminés, et dans lequel la séquence modifiée a un degré d'homologie de 95% ou davantage avec la séquence soulignée sur la Figure 3.

6. Molécule d'ADN qui code pour un polypeptide tel que défini dans l'une quelconque des revendications 1 à 5.

7. Molécule d'ADN selon la revendication 6, qui a la séquence allant du nucléotide 64 au nucléotide 1128 représentée sur la Figure 2.

8. Vecteur qui incorpore une molécule d'ADN telle que définie dans la revendication 6 ou 7 et qui est capable, lorsqu'il est disposé dans un hôte approprié, d'exprimer ledit polypeptide.

9. Vecteur selon la revendication 8, qui est un plasmide.

10. Plasmide pFC 138 représenté sur la Figure 4.

11. Plasmide pFC 164 représenté sur la Figure 4.

12. Hôte transformé par un vecteur ou un plasmide tel que défini dans l'une quelconque des revendications 8 à 11.

13. Hôte selon la revendication 12 qui est une lignée de cellules de mammifères.

14. Hôte selon la revendication 12 qui est une bactérie.

15. Procédé pour la préparation d'un polypeptide tel que défini dans l'une quelconque des revendications 1 à 5, lequel procédé comprend la culture d'un hôte transformé tel que défini dans l'une quelconque des revendications 12 à 14 dans des conditions telles que ledit polypeptide soit exprimé.

16. Composition pharmaceutique comprenant un support ou diluant pharmaceutiquement acceptable et, comme principe actif, un polypeptide tel que défini dans l'une quelconque des revendications 1 à 5.

17. Polypeptide tel que défini dans l'une quelconque des revendications 1 à 5, destiné à être utilisé comme antagoniste pour le bFGF humain ou l'aFGF humain.
